# EUROPEAN PATENT APPLICATION

(11) **EP 1 837 457 A1**
(43) Date of publication of application: **26.09.2007**
(21) Application number: 05813980.9
(22) Date of filing: 25.11.2005
(51) Int. Cl.: E04F 15/022

(54) **A MAGNETIC HYGIENICALLY STRENGTHENING WOOD FLOOR**

(30) Priority: 14.01.2005 CN 200510020173
(71) Applicant: Sichuan Shengda Forest Products Industry Group Co., Chengdu, Sichuan 610016 (CN)
(72) Inventor: YU, Gang, 26 Floor Shibaida Bldg., Chengdu, Sichuan 610016 (CN); YI, Shu, 26 Floor Shibaida Bldg., Chengdu, Sichuan 610016 (CN)
(74) Representative: Lind, Urban Arvid Oskar
(86) International application number: PCT/CN2005/001998
(87) International publication number: WO 2006/074588

(57) **Abstract**

The present invention relates to a magnetic health-care laminated wooden floor, which comprises, connected in sequence, an abrasion-resistant layer 1, a decorative layer 2, a core layer 3, a balance layer 4, and a magnetic sheet 5 bonded on the bottom of balance layer 4. The magnetic induction strength on the surface of the floor is 1 to 20 Gs. The magnetic sheet comprises A: magnetic powder 100, B: polymer 5 to 30, and C: additive 0.1 to 5 parts by weight. The preparation method comprises the steps of: (1) hot pressing an abrasion-resistant layer 1, a decorative layer 2, a core layer 3 and a balance layer 4 into a composite, then cutting, planing and milling the composite around four edges into base boards; and (2) using an appropriate adhesive to bond a magnetic sheet 5 on the bottom of balance layer 4. The floor of the present invention can improve the indoor environment weakened in geomagnetic field, and realize the health-care functions of regulating lipid metabolism and improving microcirculation by virtue of the action of the additional magnetic field on the acupoints of human foot, and can also lower footstep noises to quiet indoor environment.

## Description

### TECHNICAL FIELD OF THE INVENTION

The present invention relates to indoor flooring decoration materials, particularly to a magnetic health-care laminated wooden floor, a method for preparation and use of the same.

### BACKGROUND OF THE INVENTION

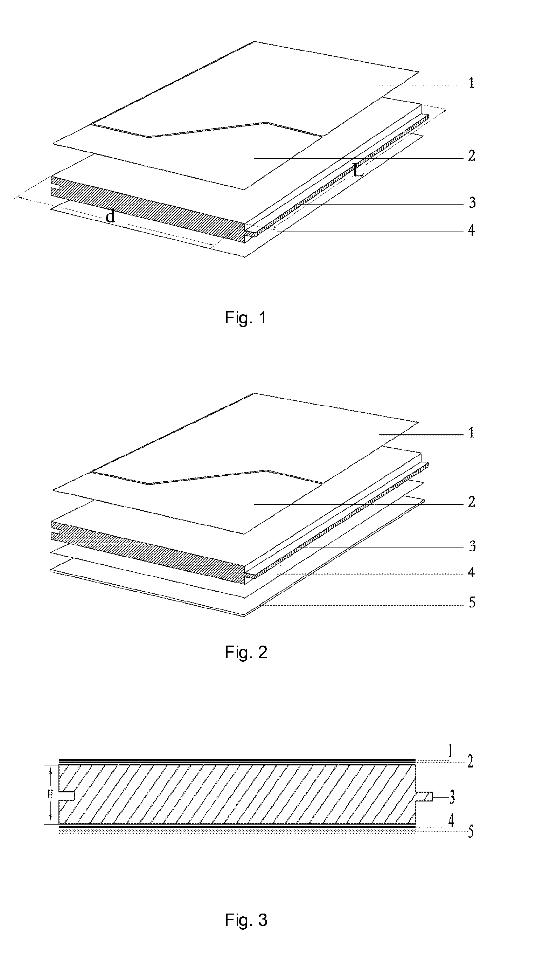

The indoor decoration materials are one of the most important parts of living environment. In recent years, the laminated wooden floor (usually-called as strengthening wooden floor, Figure 1), which is made by laminating an abrasion-resistant layer 1 comprising Aluminium Oxide (Al₂O₃) and a decorative layer 2 impregnated with melamine-formaldehyde (MF) resin (1 and 2 are blended into the outer surface), a core layer 3 of middle-, high-density fiberboard or particleboard in special form, and a balance layer 4 as under-layer, has become popular flooring material in the market due to its outstanding advantages of abrasion and scratch resistance, burn resistance, good decorative effect as a natural sensation of wood texture, easy to clean and impact resistance etc., and is widely used as a high-grade decorative material of indoor flooring in public buildings and private residences.

Though the laminated wooden floor can beautify indoor environment, its function is so monotonous, and even releases organic gas such as formaldehyde during its application to endanger human health. In these days, the sustainable development of living environment coordinated with human life has become the mainstream, people are raising the requirements of indoor environment quality after decoration, which include not only beautifying the environment, safe and reliable, but also benefiting to human heath, and even taking the effect of health protection.

The geomagnetic field is generated as the movement of electric conductive fluid in the Earth's outer core, so that a strong magnetic force is sent out continuously from the centers close to the North and South Poles to make the ground magnetic field strength reach up to 0.35 ~ 0.7 Gs. Various organisms have to adapt to the Earth's environment for survival competition. Thus, the geomagnetic field which is in widespread existence will influence on the evolution and existence of organisms.

It has been found by modern studies that the magnetic Fe₃O₄ nanoparticles synthesized by internal tissues are presented in the living body (including the human brain), and all of the main organs maintaining the life activity of human body, such as brain, heart and lung etc., may send out a very weak magnetic field. Namely, human beings have formed their own biological magnetic field during long-term evolution. So the magnetic characteristics and their changes of human body itself and environment (mainly is the geomagnetic field) will have a significant effect on human health. Just like sunlight, air and water, the geomagnetic field can't be separated from human health. Despite living in this large magnetic biosphere for a long time, it is not easy for people to concern about the geomagnetic field, because it can't be seen, touched and smelt.

However, the natural geomagnetic field is shielded indoors by various magnetic conductive materials such as reinforcing steel bar, pipe network in high-rise buildings, and the magnetic field strength is reduced by 40 ~ 50%, which thereby breaks the geomagnetic environment to which human being has long-term adapted. Therefore, the people residing or working in high-rise buildings for a long time are easier to suffer from "magnetic hunger syndrome", the clinical manifestations of which include intractable headache, insomnia, tiredness, arthralgia and the like.

China is the earliest country of discovering the magnetic phenomena and applying magnetic materials, a typical example is compass which is one of the four great inventions in ancient, and the history used magnetite to medical treatment is also over 2,000 years. It is showed by studying on modern biological magnetism that both the applied magnetic field and the environmental magnetic field will affect on organisms, which are the so-called magnetic biological effect. Such effect is related with the strength, uniformity and frequency of magnetic field, and also with the species of organisms, the location and time subjected to a magnetic field etc.

Both at home and abroad, the studies on magnetic flooring are mainly focused on how to achieve the purposes in terms of convenience to construct, easy to exchange and providing special decoration, just like those disclosed in the Chinese patents CN2437786Y, CN2455829Y and US5271200. Although people had tried to develop a magnetic floor to improve the phenomena that indoor geomagnetic field was weakened by various reasons, for example, CN2154883Y disclosed an indoor magnetic floor comprising a support layer made of plastic, rubber or silicate and a magnetic layer on one side of which the magnetic material was pressed or adhered, which only provided a magnetic field strength of 27 ~ 56 A/m (0.339 ~ 0.703 Gs), equivalent to the magnetic field strength of ground. What's more, CN1544222A disclosed a method as follows: firstly, some magnets were fixed on a high-density substrate by using a universal adhesive, and pressed between a decorative layer and the high-density substrate, and then a abrasion-resistant layer was molded on the above-resulted laminate to form convex magnets, which take the action of foot massage and magnetic therapy. The patent application did not define the type or composition of the magnetic material and the magnetic field strength, let alone verifying the health-care effect and mechanism. In addition, the process is complicated, the abrasion-resistant layer and decorative layer impregnated by melamine-formaldehyde resin will become very brittle, so this method can easily lead to the brittle fracture of abrasion-resistant layer and decorative layer, and can't be used in large-scale industrial production.

Therefore, it has significance in practical application that how to develop a magnetic health-care laminated wooden floor featured with simple preparation method (easy to carry out large-scale production based on without changing the existing process and equipments for preparing the laminated wooden floor), easy to select materials, and suitable cost.

### DETAILED DESCRIPTION OF THE INVENTION

One of the objects of the present invention is to develop a laminated wooden floor with additional magnetic field, so as to overcome the disadvantages of the existing indoor flooring, improve the indoor environment weakened in geomagnetic field, and realize the health-care functions of adjusting blood lipid metabolism and improving microcirculation by virtue of the action of the additional magnetic field on the acupuncture points of human foot, and lower the noises caused by walking on flooring to quiet indoor environment as well.

Another object of the present invention is to provide a method for peparing a laminated wooden floor with additional magnetic field.

Still another object of the present invention is to provide the use of the laminated wooden floor with additional magnetic field for adjusting the levels of cholesterol and triglyceride in human blood.

A further object of the present invention is to provide the use of the laminated wooden floor with additional magnetic field for lowering footstep noises.

Specifically, the present invention is realized by the following technical solutions:
The magnetic health-care laminated wooden floor of the present invention comprises, connected in sequence, with an abrasion-resistant layer 1, a decorative layer 2, a core layer 3 and a magnetic sheet 5 (Figures 2 and 3).
The magnetic induction strength on the surface of the floor herein is 1 ~ 20 Gs.
There is a balance layer 4 connected on the top of magnetic sheet 5.
The magnetic sheet 5 herein comprises the following components, based on parts by weight:

| | | |
|---|---|---|
| A: | magnetic powder | 100 |
| B: | polymer | 5~30 |

wherein, the magnetic powder is ferrite series or Nd-Fe-B series permanent magnetic powder, and the polymer is one or more kinds selected from the group consisting of rubbers, thermoplastic and thermosetting macromolecular resins. The rubber is preferably selected from the group consisting of nitrile rubber, chloroprene rubber or ethylene-propylene rubber. The thermoplastic macromolecular resin is preferably selected from the group consisting of chlorinated polyethylene, polyethylene, polyvinyl chloride, polypropylene, ethylene-acrylate copolymer or ethylene-vinyl acetate copolymer. The thermosetting macromolecular resin is preferably selected from the group consisting of epoxy resin, phenolic resin or unsaturated polyester.

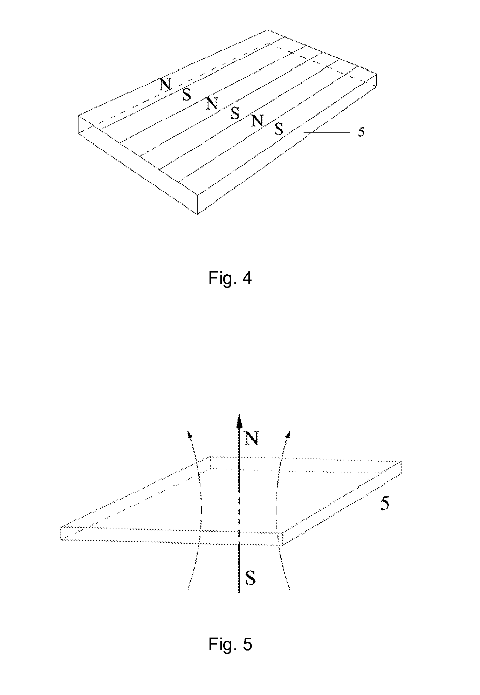

According to the difference of polymer to be used, the magnetic sheet 5 used herein may further comprises an additive C, which is one or more kinds selected from the group consisting of coupling agent, curing agent, curing accelerant, cross-linking agent, plasticizer, antioxidant, lubricant and stabilizer. The additive C is usually used in an amount of 0.1 ~ 5 parts by weight. The magnetic sheet formed according to the above-mentioned formulation is magnetized by the way of single side-multiple poles arranged as S-N-S-N (Figure 4), or axial double sides-single pole arranged as S-N (Figure 5).

The method for preparing the magnetic health-care laminated wooden floor herein comprises the steps as follows:
(1) hot pressing an abrasion-resistant layer 1, a decorative layer 2 and a core layer 3, or an abrasion-resistant layer 1, a decorative layer 2, a core layer 3 and a balance layer 4 into a composite , then cutting, planing and milling the composite around four edges into a base board; and
(2) using a suitable adhesive to bond a magnetic sheet 5 on the bottom of the core layer 3 or balance layer 4.

The present invention further comprises the following pre-treatment steps of:
(a) impregnating the abrasion-resistant layer 1, the decorative layer 2 and the balance layer 4 with a melamine-formaldehyde resin, separately; and
(b) blending various components of the magnetic sheet to be uniform, and processing them after crushing or granulating into a sheet, and then magnetizing the sheet with a magnetizing machine. Thereafter, the step (1) or (2) described as the above preparing method is performed.

The magnetic health-care laminated wooden floor in the present invention is obtained by assembling a magnetic layer with a certain magnetic field strength and direction into the structure of an ordinary laminated wooden floor, so as to provide an appropriate additional magnetic field to improve the indoor environment weakened in geomagnetic field, and enable people naturally to enjoy the magnetic therapy and health-care at work or home.

Metabolism is the basis of life activity. The concentration of lipid in plasma of normal human bodies or animals has to maintain relative stableness, which is also to say that lipid metabolism should keep balance. Enzyme is a special protein, and also is a catalyst for in vivo metabolism. All the biochemical reactions in vivo are carried out under the catalysis of enzymes.

Under the action of an additional magnetic field designed in the present invention, the activity of 7α-hydroxylase, which is a rate-limiting enzyme for decomposing the cholesterol, may be increased to thereby raise the organism's own ability of decomposing cholesterol. Therefore, the organisms, when absorbing excessive cholesterol from foods, can more easily decompose and excrete cholesterol under the action of the additional magnetic field, and restore the state of relative balance within a certain period of time, to thereby maintain the normal level of blood lipid. This is the health-care effect of regulating lipid metabolism.

The main task of blood circulation is to transport various substances and metabolites, and then enable metabolism to proceed normally. Blood microcirculation is the blood circulation between arteriole and veinule. Once there are obstacles in microcirculation function, will lead to physiological dysfunction, and result in various diseases such as hyperlipidemia, especially atherosclerosis, coronary artery disease and cerebrovascular disease.

The increase in the level of blood lipid is an important factor to cause microcirculation obstacles. The mechanism is when the level of blood lipid rises, large amount of cholesteryl ester will accumulate until fill in the whole blood cells, such cells are called as foam cells, which can easily deposit on the arterial wall, make the artery narrow and its wall rough, increase the resistance to blood flow, so that the rate of blood flow will be slowered and the blood microcirculation will be inhibited. As the results, blood is easy to be extravasated and even be stasis in the capillary vessels, thereby cause the microcirculation obstacles.

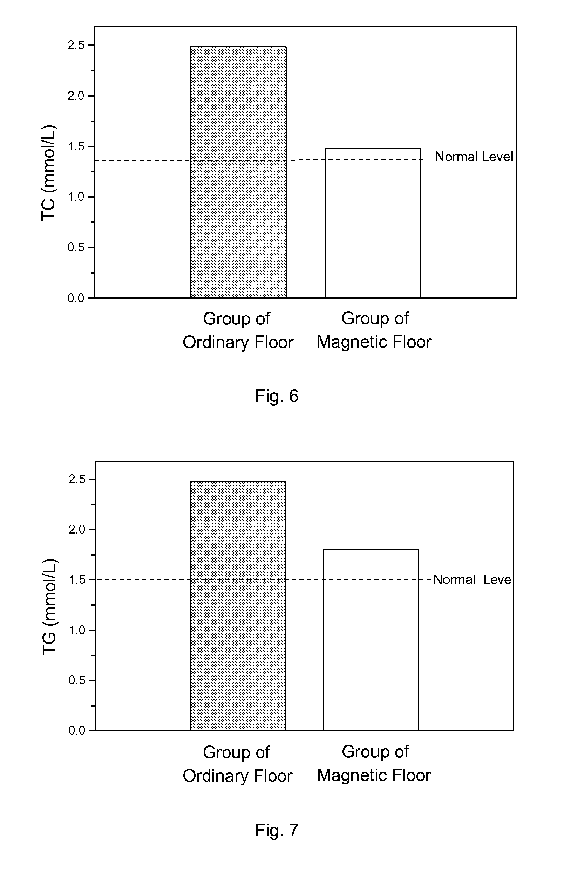

Sichuan Institute of Natural Pharmaceutical (obtained GLP certification awarded by State Food and Drug Administration) and Testing and Analysis Center of Huaxi Public Health College, Sichuan University (health food test organization designated by State Ministry of Health, obtained CMA certification) were commissioned to carry out the functional animal tests. The results showed that the content of total cholesterol (TC) in the blood of rats assigned in the group of magnetic health-care laminated wooden floor of the present invention was remarkably lower than that in the contrasting group of ordinary laminated wooden floor (P < 0.01, Figure 6); and the content of triglyceride (TG) in the blood of rats assigned in the group of magnetic health-care laminated wooden floor of the present invention was obviously lower than that in the contrasting group of ordinary laminated wooden floor (P < 0.05, Figure 7). It is indicated that the magnetic health-care laminated wooden floor of the present invention can obviously inhibit the rise of blood lipid including cholesterol and triglyceride in the tested rats, which suggests that the magnetic health-care laminated wooden floor of the present invention has the health-care functions of regulating lipid metabolism and improving microcirculation, and takes a positive role for preventing various hyperlipidemia and cardiovascular diseases caused by microcirculation obstacles.

According to Chinese medicine, meridians and collaterals are regarded as a network of passages, through which vital energy and blood of human body circulate, and spread all over the body to connect all tissues and organs into a whole, so that the bodily functions are regulated. Feet are the second heart of human body and very important to human health. Among the twelve meridians, the stomach meridian of foot-yangming, gallbladder meridian of foot-shaoyang, urinary bladder meridian of foot-taiyang, lung meridian of foot-taiyin, liver meridian of foot-jueyin and kidney meridian of foot-shaoyin, all of which begin from feet and end on feet, can directly or indirectly control the physiological actions of various entrails and organs of human body.

It has been proved by modern biological magnetism that acupuncture points are the sensitive points of human body's magnetic field, of which pubic region is the center, meridians and collaterals are the passages through which electromagnetism conducts. A magnetic field applied at a corresponding acupoint will be transferred through the meridians and collaterals to regulate the bodily functions, thereby the purpose of health promotion (health care) or therapy is achieved.

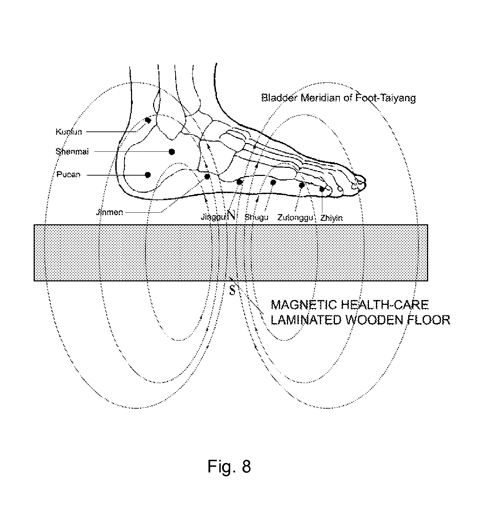

Just based on the principle of magnetic therapy through foot acupoints, the health-care design for magnetic health-care laminated wooden floor of the present invention is realized by virtue of the action of an additional magnetic field on the human foot acupoints (Figure 8).

The impact sound (belonged to solid sound) generated by walking and jumping with ordinary leather shoes or high-heeled shoes on flooring is the primary indoor noise, which is generally difficult to be insulated due to its low attenuation propagated through the structure of flooring. The Environmental Test Center of Architectural Physics, Qinghua University was commissioned to measure the footstep noise generated by walking with leather shoes or high-heeled shoes on the flooring.

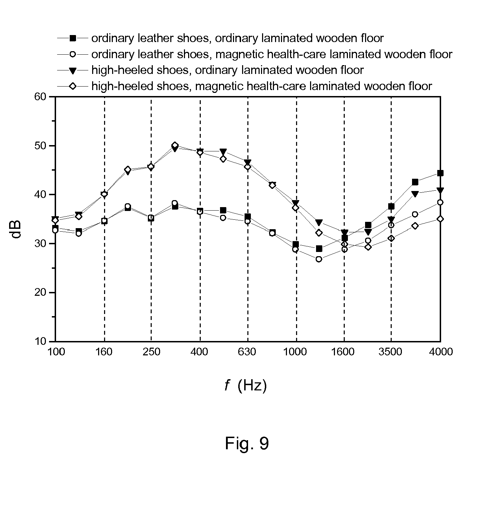

The test results showed (Figure 9) that compared with ordinary laminated wooden floor, the footstep noise generated by walking with ordinary leather shoes on the magnetic health-care laminated wooden floor was reduced by 4 dB, which was equivalent to a 60% reduction of the noise energy; the footstep noise generated by walking with high-heeled shoes was reduced by 2 dB, which was equivalent to a 37% reduction of the noise energy. From the acoustic physiology point of view, human ears can obviously hear the reduction in environmental noises when which is reduced by ≥ 2 dB. Therefore, the magnetic health-care laminated wooden floor of the present invention has an obvious effect to lower the noise and quiet the environment, and benefits to human health.

The safety limits of human exposure to constant magnetic field set forth by domestic and overseas standards are 10 mT for long-term systemic exposure, and 100 mT for long-term limbs exposure. The constant magnetic induction strength on the surface of magnetic health-care laminated wooden floor of the present invention is ≤ 2 mT, so it is safe and reliable.

In addition, Maxwell's electromagnetic theory shows that waves can be generated when electromagnetic field changes with time. Since the magnetic field strength of a constant magnetic field does not change with time (dH/dt = 0), the constant magnetic field attached to the magnetic health-care laminated wooden floor of the present invention won't cause electromagnetic interference in mobile phone, TV and so on.

To sum up, the present invention takes into consideration the deficiencies of indoor flooring, particularly concerning with the laminated wooden floor, of which a magnetic sheet with a certain magnetic field strength and direction is laminated or assembled on the bottom, so the surface of which is endowed with a certain magnetic induction strength that is safe for human body and indoor environment, without affecting its surface properties (decoration as a natural sensation of wood texture, abrasion resistance and burn resistance), and comprehensive properties of physics and chemistry, so as to improve the indoor environment weakened in geomagnetic field, and realize the health-care functions of regulating lipid metabolism and improving microcirculation by virtue of the action of the additional magnetic field on human foot acupoints. What's more, the magnetic sheet assembled can also lower footstep noises and play a role to quiet indoor environment (the acoustic performance is better than that of the laminated wooden floor back-pasted with foam or cork which is popular in market).

### BRIEF DESCRIPTION OF THE DRAWINGS

Figure 1 illustrates the structure of ordinary laminated wooden floor.
Figure 2 illustrates the structure of the magnetic health-care laminated wooden floor.
Figure 3 illustrates the section of the magnetic health-care laminated wooden floor.
Figure 4 illustrates the single side-multiple poles magnetic path of magnetic sheet.
Figure 5 illustrates the axial double sides-single pole magnetic path of magnetic sheet.
Figure 6 describes the effect of the magnetic health-care laminated wooden floor on inhibiting the rise of cholesterol in blood of tested rats.
Figure 7 describes the effect of the magnetic health-care laminated wooden floor on inhibiting the rise of triglycerides in blood of tested rats.
Figure 8 illustrates the mechanism of the magnetic health-care laminated wooden floor acting on human feet.
Figure 9 describes the spectrum of noises caused by walking on the magnetic health-care laminated wooden floor.

### DESCRIPTION OF THE PREFERRED EMBODIMENTS

The present invention is further described by the following examples and figures. To be clear, these examples are not used to limit the protection scope of the present invention. The protection scope of the present invention is determined by the attached claims.

An ordinary laminated wooden floor, with a structure shown in Figure 1, was used as contrastive sample in the present invention, and prepared by the steps of firstly impregnating an abrasion-resistant layer 1, a decorative layer 2 and a balance layer 4 separately with a melamine-formaldehyde resin, then hot pressing them into a composite, finally cutting, planing and milling the composite around four edges into final products. The magnetic health-care laminated wooden floor of the present invention, with a structure shown in Figure 2, was prepared by bonding a magnetic sheet 5 on the bottom of balance layer 4 with an appropriate adhesive. There is no special restriction to the size of flooring and the adhesive used in the invention. The magnetic sheets used in the examples were made by blending the following components to be uniform, processing them after crushing or granulating into a sheet, and then magnetizing the sheet by a magnetizing machine. The performance parameters of the magnetic sheets are listed as follows:

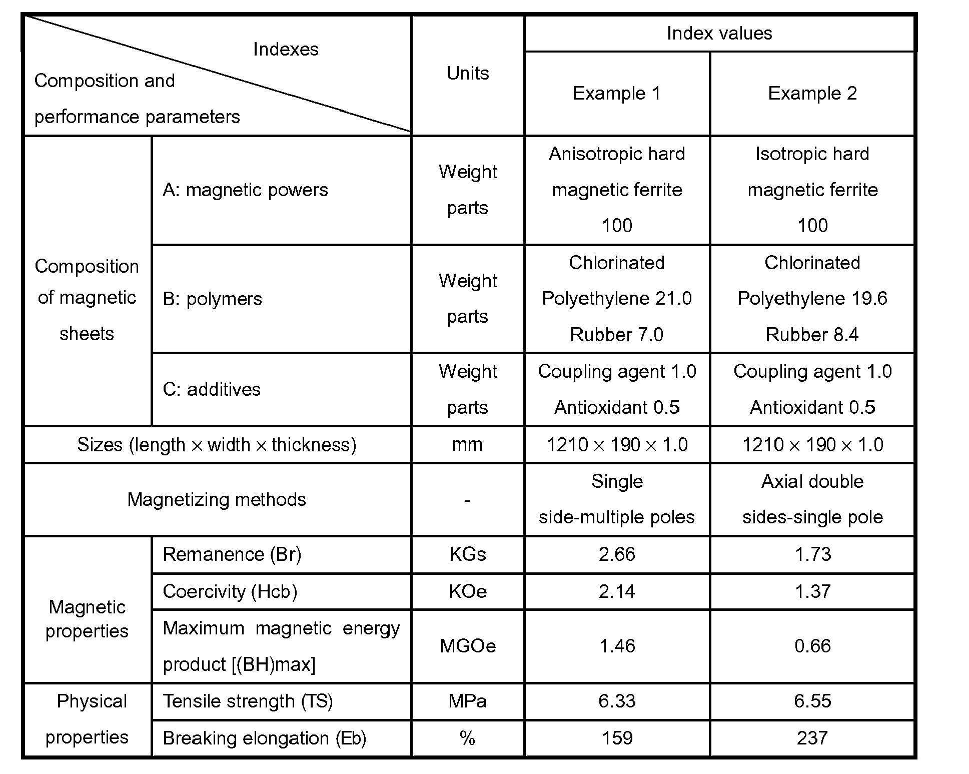

The properties of floors prepared according to the above methods are listed as follows:

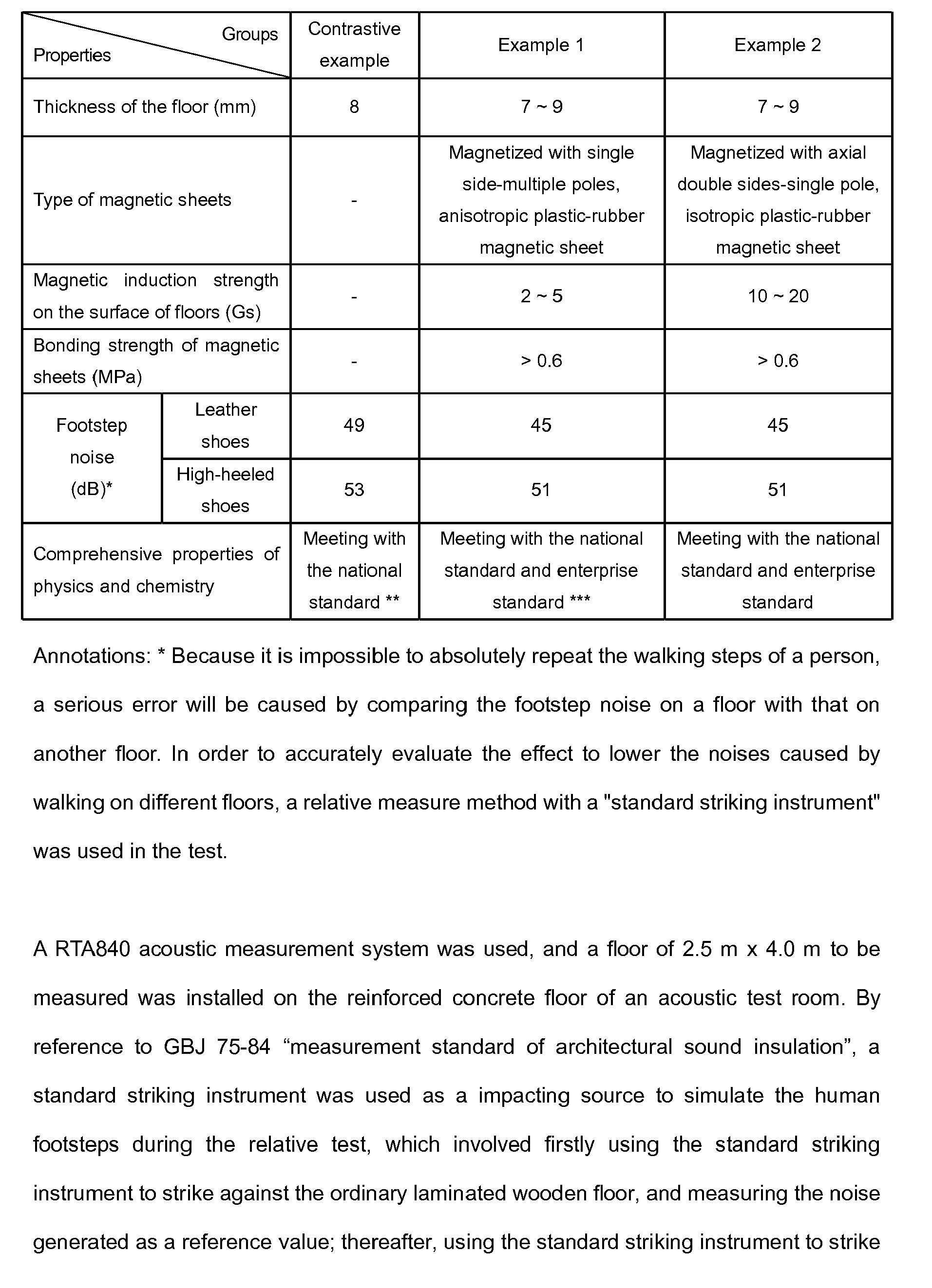

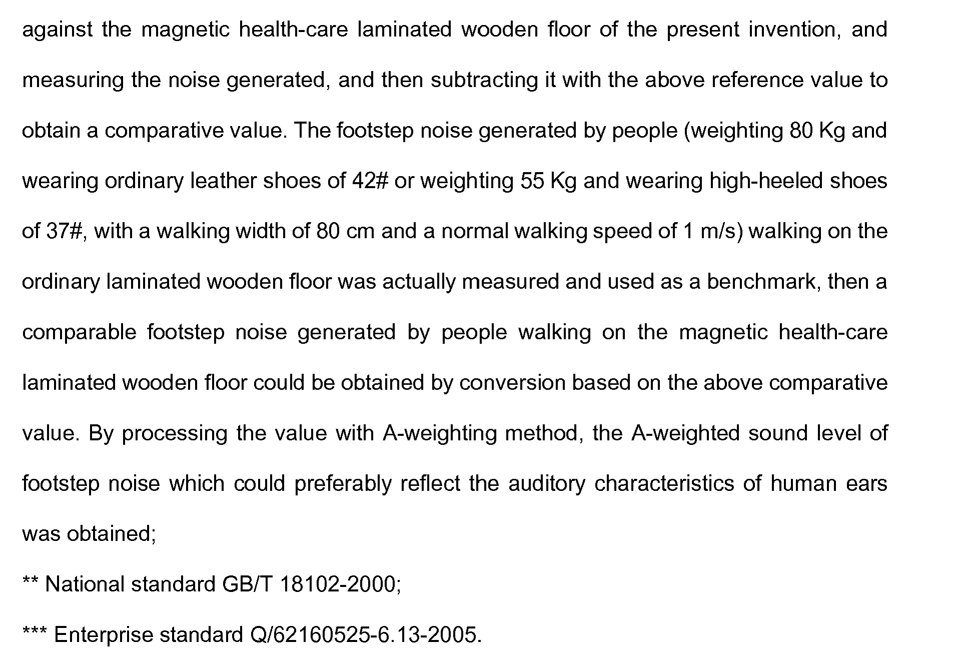

Referring to "Technical Standards of Health Food Test and Evaluation" (edition in 2003) issued by the State Ministry of Health, the rats to be tested were divided into a contrastive example group and an example group, including 10 ~ 15 ones in each group. The rats in the example group were fed by gavages with a high fat forage comprising cholesterol, cholic acid, propylthiouracil and grease (one time per day, at a dose of 1 ml/100g of the rat body weight) on the magnetic health-care laminated wooden floor, while the rats in the contrastive example group were fed with the same forage on the ordinary laminated wooden floor in the absence of an additional magnetic field. After about 20 days, the rats were anaesthetized with ethyl ether and 4 ml blood was taken from the abdominal aorta, of which 3 ml was anticoagulated by heparin sodium to use for the measurement of whole blood viscosity and plasma viscosity, and 1 ml was centrifuged at the rate of 3,000 rpm to pick-up the serum, and then the contents of TG, TC, HDL-C and LDL-C in the serum were measured, and the ratios of HDL-C and LDL-C based on the total cholesterol (HDL-C/TC, LDL-C/TC) were calculated. The results were subjected to t-test.

Based on the animal experiments, the magnetic biological effects of examples and contrastive examples in the present invention were evaluated as follows:
The effect of magnetic health-care laminated wooden floor of the present invention on inhibiting the rise of blood lipid in the tested rats (*x̅±SD*):

| Groups | TC (mmol/L) | TG (mmol/L) | LDL-C (mmol/L) | HDL-C/TC (%) | LDL-C/TC (%) |
|---|---|---|---|---|---|
| Contrastive example 1 | 2.490 ± 0.907 | 0.675 ± 0.114 | 1.604 ± 0.852 | 24.8 ± 4.9 | 62.1 ± 7.8 |
| Example 1 | 1.481 ± 0.303** | 0.644 ± 0.134 | 0.741 ± 0.321** | 31.3 ± 7.5* | 48.3 ± 11.7** |

| | | | | | |
|---|---|---|---|---|---|
| Annotations: * Compared to contrastive example with t-test, P < 0.05; ** Compared to contrastive example with t-test, P < 0.01. | | | | | |

The effect of magnetic health-care laminated wooden floor of the present invention on inhibiting the rise of blood lipid in the tested rats (*x̅*±*SD*):

| Groups | TC (mmol/L) | TG (mmol/L) | LDL-C (mmol/L) | HDL-C (mmol/L) |
|---|---|---|---|---|
| Contrastive example 2 | 2.89 ± 0.51 | 2.48 ± 0.97 | 0.85 ± 0.39 | 0.92 ± 0.17 |
| Example 2 | 2.72 ± 0.29 | 1.81 ± 0.79* | 1.08 ± 0.39 | 0.82 ± 0.10 |

| | | | | |
|---|---|---|---|---|
| Annotation: * Compared to contrastive example with t-test, P < 0.05. | | | | |

The effect of magnetic health-care laminated wooden floor of the present invention on inhibiting the rise of blood viscosity of the tested rats (*x̅±SD*):

| Groups | Whole blood viscosity (20 °C ,cps) | | | Plasma viscosity (20 °C, cps) |
|---|---|---|---|---|
| | Shear rate 200 (1/s) | Shear rate 30 (1/s) | Shear rate 5 (1/s) | |
| Contrastive example 1 | 6.257 ± 0.814 | 8.379 ± 1.134 | 14.644 ± 2.082 | 1.706 ± 0.024 |
| Example 1 | 5.917 ± 0.465 | 8.006 ± 0.667 | 14.206 ± 1.396 | 1.678 ± 0.015* |

| | | | | |
|---|---|---|---|---|
| Annotation: * Compared to contrastive example with t-test, P < 0.05. | | | | |

## Claims

1. A magnetic health-care laminated wooden floor, **characterized in that** the floor comprises, connected in sequence, an abrasion-resistant layer 1, a decorative layer 2, a core layer 3 and a magnetic sheet 5; and the magnetic induction strength on the surface of the floor is 1 to 20 Gs.

2. The magnetic health-care laminated wooden floor according to claim 1, **characterized in that** a balance layer 4 is connected on the top of magnetic sheet 5.

3. The magnetic health-care laminated wooden floor according to claim 1, **characterized in that** the magnetic sheet 5 comprises the following components, based on parts by weight:
| | | |
|---|---|---|
| A: | magnetic powder | 100 |
| B: | polymer | 5 to 30 |
wherein, the magnetic powder is ferrite series or Nd-Fe-B series permanent magnetic powder, and the polymer is one or more kinds selected from the group consisting of rubbers, thermoplastic and thermosetting macromolecular resins.

4. The magnetic health-care laminated wooden floor according to claim 3, **characterized in that** the magnetic sheet further comprises 0.5 to 5 parts by weight of additive C which is one or more kinds selected from the group consisting of coupling agent, curing agent, curing accelerant, cross-linking agent, plasticizer, antioxidant, lubricant and stabilizer.

5. The magnetic health-care laminated wooden floor according to claim 1,
**characterized in that** the formed magnetic sheet is magnetized by the path of single side-multiple poles arranged as S-N-S-N, or axial double sides-single pole arranged as S-N.

6. The magnetic health-care laminated wooden floor according to claim 1, **characterized in that** the formed magnetic sheet is magnetized by the path of axial double sides-single pole arranged as S-N.

7. A method of manufacturing the magnetic health-care laminated wooden floor according to any one of claims 1 to 6, comprising the steps of:
(1) hot pressing an abrasion-resistant layer 1, a decorative layer 2 and a core layer 3, or an abrasion-resistant layer 1, a decorative layer 2, a core layer 3 and a balance layer 4 into a composite, then cutting, planing and milling the composite around four edges into a base board; and
(2) using a suitable adhesive to bond a magnetic sheet 5 on the bottom of core layer 3 or balance layer 4.

8. The method according to claim 7, further comprising the following pre-treatment steps of:
(a) impregnating the abrasion-resistant layer 1, the decorative layer 2 and the balance layer 4 with a melamine-formaldehyde resin, separately; and
(b) blending various components of the magnetic sheet to be uniform, and processing them after crushing or granulating into a sheet, and then magnetizing the sheet with a magnetizing machine.

9. Use of the magnetic health-care laminated wooden floor according to any one of claims 1 to 6 for regulating the contents of cholesterol and triglyceride in human blood.

10. Use of the magnetic health-care laminated wooden floor according to any one of claims 1 to 6 for lowering and quieting the noises.
